(19) Europäisches Patentamt
European Patent Office
Office européen des brevets

(11) Publication number: **0 446 772 A1**

(12) **EUROPEAN PATENT APPLICATION**

(21) Application number: **91103405.6**

(51) Int. Cl.5: **C07C 251/04**, //C23C16/18

(22) Date of filing: **06.03.91**

(30) Priority: **12.03.90 US 491909**

(43) Date of publication of application: **18.09.91 Bulletin 91/38**

(84) Designated Contracting States: **AT BE CH DE FR GB IT LI SE**

(71) Applicant: **AIR PRODUCTS AND CHEMICALS, INC.**
**7201 Hamilton Boulevard**
**Allentown, PA 18195-1501(US)**

(72) Inventor: **Norman, John Anthony Thomas**
**3032 North 4th Street**
**Whitehall, PA 18052(US)**

(74) Representative: **Kador & Partner**
**Corneliusstrasse 15**
**W-8000 München 5(DE)**

(54) **Fluorinated beta-ketoiminato metal complexes.**

(57) Fluorinated β-ketoimine ligands and highly volatile β-ketoiminato metal complexes of the ligands are synthesized by silylating a fluorinated β-diketone to form a silylenolether, and subsequently reacting the silylenolether with a primary diamine to form the desired ligand having the structural formula:

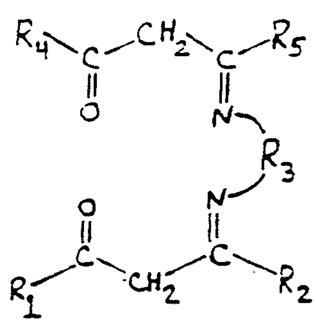

wherein $R_1$, $R_2$, $R_4$ and $R_5$ are independently linear or branched perfluorinated, $C_1$-$C_8$ alkyl groups and $R_3$ is any organic functionality such as a $C_1$-$C_8$ alkylene, phenylene or hydroxyalkylene group, all of which can be partially or fully fluorinated. The corresponding metal complex is formed by treating the ligand with a metal halide.

EP 0 446 772 A1

## TECHNICAL FIELD

The present invention relates to fluorinated organic ligands and volatile metal complexes formed from such ligands.

## BACKGROUND OF THE INVENTION

In the electronics industry there is a growing need for volatile sources of different metals to be used in the chemical vapor deposition (CVD) of metallic films, metal oxide films, metal silicide films, and the like. The key property required for such metal sources is that they readily evaporate or sublime to give a metal containing vapor or gas which can be decomposed in a controlled manner to deposit a film onto a target substrate. Examples of such materials which are commonly utilized in the microelectronics industry in the preparation of printed circuits and semiconductor devices include the complex $H_3SiCo(CO)_4$ complex which is pyrolyzed in the gas phase at 670-770 °K to produce CoSi and dimethylzinc (1,4 dioxane) which is reacted with hydrogen selenide at 250-550 °C to produce ZnSe. References teaching the above CVD methods are B. J. Aylett, et al in Vacuum, 35 p 435-439(1985) and P. J. Wright, et al., in a paper accepted for publication in J. of Crystal Growth, London (1986), respectively.

Known fluorinated metal complexes that are chemically stable and easily volatized into the gas phase are the perfluorinated $\beta$-diketone metal coordination compounds along with their parent $\beta$-diketone precursor ligands, represented by the formulas:

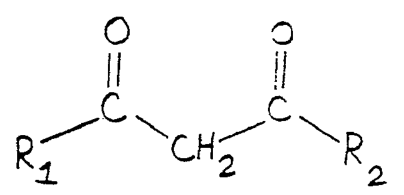

Ligand

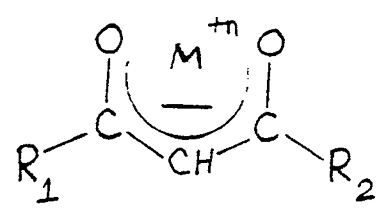

Metal Coordination Compound

wherein $R_1$ is alkyl or fluoroalkyl, $R_2$ is fluoroalkyl, and $M^{+n}$ is a metal ion capable of forming a coordination compound. The volatility and gas phase stability of these compounds have been exploited for the gas chromatographic separation of various metals, the purification of uranium and the manufacture of specialty glasses. Decomposing such metal complexes by reaction with hydrogen in the gas phase to deposit thin metal films is taught in U.S. Patent 3,356,527.

In the past, attempts have been made to condense primary amines or primary diamines with ligands similar to those having the above structure. In instances in which $R_1$ and $R_2$ are not both fluorocarbon groups, it was reported that an O atom could be replaced with a N atom from an amine by direct Schiff-base condensation between an appropriate $\beta$-diketone and an amine. Additionally, the corresponding metal complex could be synthesized by chelation to a metal ion. See A. E. Martell, et al J. Inorg. Chem. Vol. 5 pp 170-181 (1958).

As reported by Sievers, et al in J. Inorg. Nucl. Chem. Vol. 32 pp 1895-1906 (1970), ligands in which $R_1$ and $R_2$ are both perfluoralkyl and in which an oxygen has been replaced with an amine have not been obtainable. It is believed that such methods have been unsuccessful because the perfluorinated $\beta$-diketones are of such high acidity that the amine used in the reaction becomes protonated, thereby forming a salt between the amine and the $\beta$-diketone rather than forming the desired ligand. Sievers, et al do report synthesizing a ligand having the structure:

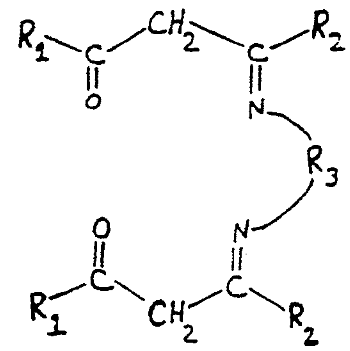

wherein $R_1$ = $R_2$ = $CF_3$ and $R_3$ = $-CH_2CH_2-$.
This ligand was reportedly synthesized by sublimation of the salt $[(CF_3C(O)CHC(O)CF_3)]_2^-$ $[NH_3\text{-}CH_2CH_2NH_3]^{+2}$. The ligand was reported to be chemically unstable and hence impossible to isolate.

In U.S. Patent 4,654,053, Sievers, et al. teach a process for separating gaseous oxygen from a multicomponent gas stream using a wide range of Schiff base metal chelates, including those containing perfluoro moieties. No special synthesis techniques are taught, however, for making the perfluoro compounds.

Charles, U.S. Patent 3,594,216 discloses a process for depositing a metallic coating on a substrate by heating the substrate and contacting it with vaporized metal-organic beta-ketoamine chelates. The metal-organic beta-ketoamines were prepared by conventional synthesis techniques. While a wide range of metal chelates are disclosed generally, none of the examples or synthesis techniques specifically use perfluorinated metal chelates.

Johnson, et al . in Journal of Fluorine Chemistry, 27 pp 371-378 (1985) reported synthesizing a ligand in which $R_1$ and $R_2$ are perfluoroalkyl and oxygen was replaced with an ammonia nitrogen. The $Cu^{+2}$ complex was also prepared and was reported to be volatile.

## BRIEF SUMMARY OF THE INVENTION

The present invention is a class of novel, β-ketoimine ligands and highly volatile metal complexes of the ligands and also a process for making the same. The β-ketoimine ligands of the present invention are those of the general structural formula:

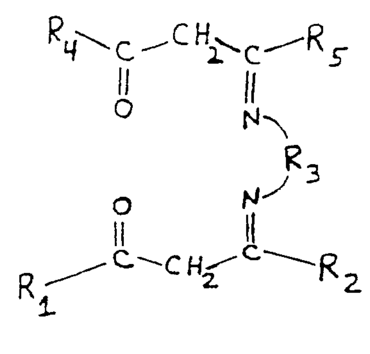

wherein $R_1$, $R_2$, $R_4$ and $R_5$ are independently linear or branched per-fluorinated, $C_1$-$C_8$ alkyl groups and $R_3$ is any organic functionality such as a $C_1$-$C_8$ alkylene, phenylene or hydroxyalkylene group, all of which can be partially or fully fluorinated.

The highly volatile β-ketoiminato metal complexes which are synthesized from these ligands have the structural formula:

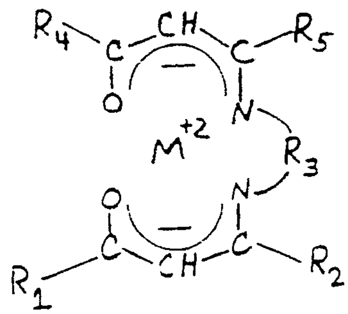

wherein $R_1$, $R_2$, $R_3$, $R_4$ and $R_5$ are as described above, and $M^{+2}$ is a divalent metal ion.

The present invention is also a process for making both the β-ketoimineligands and metal complexes described above. The ligands are synthesized by silylating a fluorinated β-diketone to form a silylenolether, and subsequently reacting the silylenolether with a primary diamine to form the desired ligand. The corresponding metal complex is formed by treating the resulting ligand with potassium methoxide followed by treatment with a halide salt of the desired metal.

## DETAILED DESCRIPTION OF THE INVENTION

The present invention is a class of heavily fluorinated β-ketoimine ligands and thermally volatilizable β-ketoiminato metal complexes of the ligands. The ligands are characterized in that they are highly fluorinated and contain oxygen and nitrogen donor atoms which can be covalently coordinated to a central metal atom to form the corresponding metal complex. This class of ligand, as well as the corresponding metal complex, are chemically stable and easily volatized into the gas phase. The high fluorine content of the complex is believed to reduce the Van der Waals forces between individual molecules and hence lower the boiling or sublimination point of the compound.

The heavily fluorinated β-ketoimine ligands of the present invention can be represented by the structural formula:

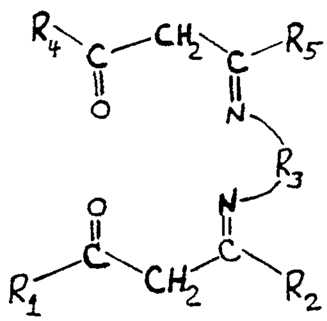

I

wherein $R_1$, $R_2$, $R_4$ and $R_5$ are independently linear or branched perfluorinated, $C_1$-$C_8$ alkyl groups and $R_3$ is any organic functionality such as a $C_1$-$C_8$ alkylene, phenylene or hydroxyalkylene group, all of which can be partially or fully fluorinated.

The highly volatile metal complexes which are synthesized from these ligands can be represented by the structural formula:

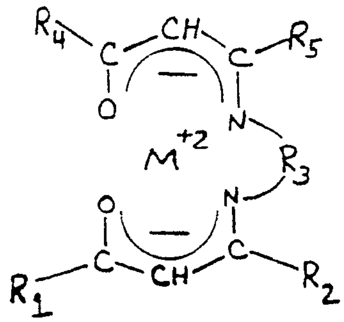

II

wherein $R_1$, $R_2$, $R_3$, $R_4$ and $R_5$ are as described above, and $M^{+2}$ is a is a divalent metal ion. These volatile complexes hold great potential for use as metal sources in Chemical Vapor Deposition (CVD) processes engaged in the art of depositing, for instance, metal films or metal oxide films.

The ligands of structure I above are synthesized by treating a fluorinated β-diketone of the formula $R_1COCH_2COR_2$ with potassium hydride under anhydrous conditions to produce a compound of the formula $R_1COCHCOR_2^- K^+$ and subsequently reacting the resultant $R_1COCHCOR_2^- K^+$ with a silylchloride such as, t-butyldimethylsilylchloride, to produce a silylenolether having the general formula:

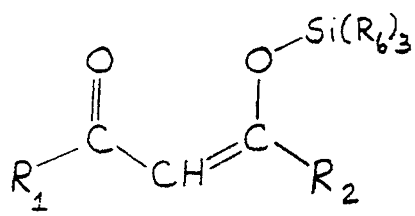

wherein $R_1$ and $R_2$ are as described above, and each $R_6$ is independently an alkyl group. The silylenolether described above is then treated with a primary diamine, $NH_2R_3NH_2$ wherein $R_3$ is as above to produce the desired β-ketoimine ligand of structural formula I.

To form the metal complex of the β-ketoimine ligand formed above, the ligand is initially treated with potassium methoxide and the resultant compound is subsequently treated with a metal halide of the formula $M^{+2}(X)_2^-$ , where X is a halogen, to form the desired highly fluorinated β-ketoiminato complex of formula II above.

The ligands of formula I produced in accordance with this invention can exist in two tautomeric forms, enol and keto, with the keto form being represented generally by formula I. Preferred ligands and metal complexes of the present invention include:

<u>Ligands.</u>

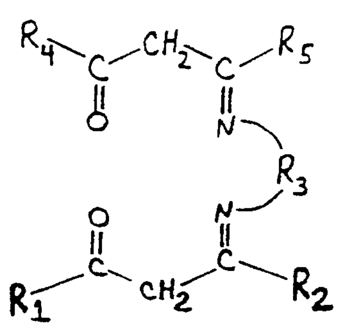

Where

$R_1 = R_2 = R_4 = R_5 = CF_3$, $R_3 = (CH_2)_2$
$R_1 = R_4 = CF_2CF_3$, $R_2 = R_5 = CF_3$, $R_3 = (CH_2)_2$
$R_1 = R_4 = CF_2CF_2CF_3$, $R_2 = R_5 = CF_3$, $R_3 = (CH_2)_2$
$R_1 = R_2 = R_4 = R_5 = CF_3$, $R_3 = (CH_2)_3$
$R_1 = R_2 = R_4 = R_5 = CF_3$, $R_3 = CH_2CH(OH)CH_2$

Complexes.

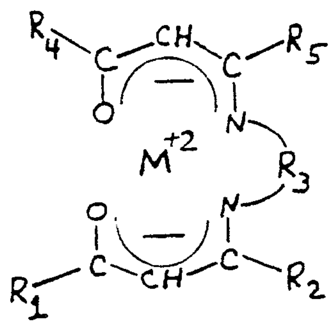

Where

$R_1 = R_2 = R_4\text{-}R_5 = CF_3$, $R_3 = (CH_2)_2$, $M^{+2} = Cu^{+2}$
$R_1 = R_2 = R_4\text{-}R_5 = CF_3$, $R_3 = (CH_2)_2$, $M^{+2} = Co^{+2}$
$R_1 = R_4 = CF_2CF_3$, $R_2 = R_5 = CF_3$, $R_3 = (CH_2)_2$ $M^{+2} = Cu^{+2}$
$R_1 = R_4 = CF_2CF_3$, $R_2 = R_5 = CF_3$, $R_3 = (CH_2)_2$ $M^{+2} = Co^{+2}$
$R_1 = R_4 = CF_2CF_2CF_3$, $R_2 = R_5 = CF_3$, $R_3 = (CH_2)_2$, $M^{+2} = Cu^{+2}$
$R_1 = R_4 = CF_2CF_2CF_3$, $R_2 = R_5 = CF_3$, $R_3 = (CH_2)_2$, $M^{+2} = Co^{+2}$

Experimental

In the following examples, temperatures are set forth uncorrected in degrees Celcius. Unless otherwise indicated, all parts and percentages are by weight.

1,1,1,5,5,5 hexafluoro-2,4-pentanedione, t-butyldimethylsilylchloride, potassium hydride, 2,2,2-trifluoroethylamine, ethylenediamine, ethanolamine, 1,3-propanediamine, 1,3-diamino-2-propanol and analine were obtained from Aldrich Chemical Co. (940 West St. Paul Ave. Milwaukee, Wis. 53233). 1,1,1,2,2,6,6,6-octafluoro-3,5-hexanedione and 1,1,1,2,2,3,3,7,7,7-decafluoro-4,6-heptanedione were obtained from Fairfield Chemical Company Inc. (P.O. Box 20, Blythewood, SC 29016).

Solvents used are HPLC grade. Tetrahydrofuran (THF) was distilled from calcium hydride under nitrogen, methanol was distilled from Mg metal under nitrogen. All operations in the preparation of the free ligands or corresponding complexes are carried out using Standard Schlenk line techniques described by D. F. Shriver, "The Manipulation of Air Sensitive Compounds" McGraw-Hill Publishing Co.

Microanalyses were performed by Schwarzkopf Microanalytical Laboratory, Woodside, NY or Research Services, Air Products and Chemicals, Inc. $^1H$, $^{19}F$ and $^{13}C$ NMR spectra were recorded using an IBM SY-200 and a Bruker WH-200 NMR spectrometer.

The chemical structure, along with both the IUPAC and abbreviated names of the ligands synthesized in the following examples are set out below. The corresponding metal complexes have the similar structure with an (H) being replaced by the metal (see formula II above). The charge on the metal complex must remain neutral, i.e., if the ligand is diprotonated one divalent metal ion such as $Cu^{+2}$ is required.

1,2-di-[4-imino-1,1,1,5,5,5-hexafluoro-2-pentanone] ethane

$(H_2)$DODECA-F[EDA]

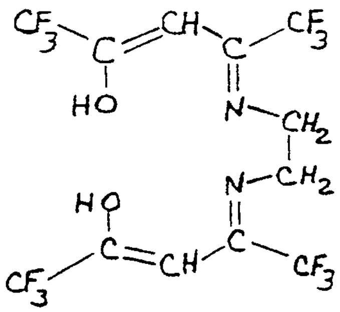

1,2-di-[5-imino-1,1,1,2,2,6,6-octafluoro-3-hexanone] ethane
($H_2$)HEXADECA-F[EDA]

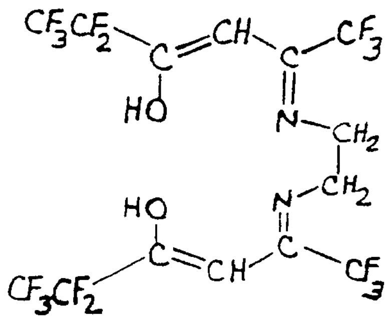

1,2-di-[6-imino-1,1,1,2,2,3,3,7,7,7-decafluoro-4-heptanone] ethane
($H_2$)EiCOSA-F[EDA]

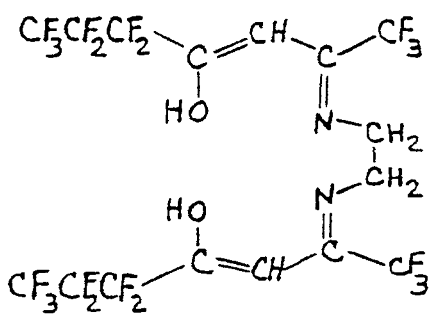

Bis[4(methylene)imino-1,1,1,5,5,5-hexafluoro-2-pentanone]methane
($H_2$)DODECA-F[PDA]

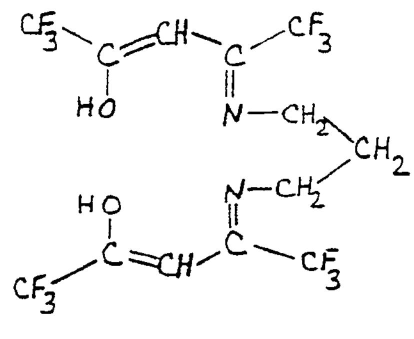

Bis[4(methylene)imino-1,1,1,5,5,5-hexafluoro-2-pentanone]methanol (H2)DODECA-F[POA]

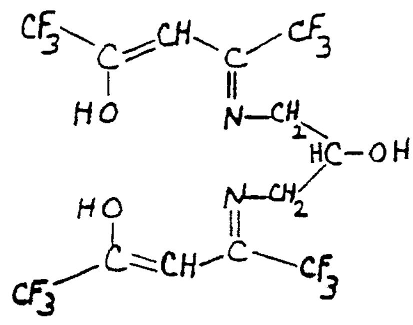

Example 1

Synthesis of the silylenolethers of perfluorinated β-diketones

The following represents a generic synthesis for the preparation of:

(i) 4-(t-butyldimethylsiloxy)-1,1,1,5,5,5-hexafluoro-3-penten-2-one from 1,1,1,5,5,5-hexafluoro-2,4-pentanediane.

(ii) 4-(T-butyldimethylsiloxy)-1,1,1,5,5,6,6,6 octafluoro-3-hexen-2-one(and its isomer 2-(t-butyldimethylisiloxy)-1,1,1,5,5,6,6,6-octafluoro-2-hexen-4-one) from 1,1,1,5,5,6,6,6-octafluoro-2,4-hexane dione.

(iii) 4-(t-butyldimethylsiloxy)-1,1,1,5,5,6,6,7,7,7-decafluoro-3-hepten-2-one (and its isomer 2-(t-butyldimethylsiloxy)-1,1,1,5,5,6,6,7,7,7-decafluoro-2-hepten-4-one) from 1,1,1,5,5,6,6,7,7,7-decafluoro-2-hepten-4-one.

Potassium hydride (20.0 g, 0.5 moles) is charged into a solid addition funnel which is fitted to a 1.01 reaction flask; the latter is also fitted with a rubber septum, an inlet for nitrogen, and a magnetic stir bar. Under an atmosphere of dry nitrogen THF (500 ml) is added to the flask which is subsequently cooled to -78°C. The perfluoro β-diketone (0.5 moles) is then added by syringe to the stirred THF at approx. 0.5 ml/min while also slowly adding potassium hydride at such a rate that it is consumed without an excess accumulating in the reaction flask. After adding all the reagents, the reaction is left to stir at room temperature until all traces of hydride are digested (up to 18 hrs). A reflux condenser and an addition funnel charged with t-butyldimethylsilylchloride (75.36 g. 0.5 moles) are fitted to the reaction flask, 150 ml THF is run into the addition funnel to dissolve the silylchloride. This solution is added dropwise over 30 mins to the stirring reaction mixture after which it is refluxed for 18 hrs. During this time a thick white precipitate of potassium chloride forms. The mixture is then filtered under nitrogen to give a pale brown or yellow filtrate. Approximately 500 ml of THF is then distilled off under nitrogen and the resulting concentrated silylenol ether solution left to cool, thereby precipitating further potassium chloride. This solution is then filtered as before then distilled under nitrogen to give the silylenolether as a moisture sensitive pale yellow liquid.
NOTE: Care must be taken to not heat the distillation flask too near to dryness. As the residual brown colored liquid in the distillation flask evaporates down to ~30 ml it may start to evolve thick yellow-grey

fumes prior to rapidly decomposing and producing a surge of pressure within the apparatus.

It is noted that two silylenolether isomers form when starting with unsymmetrical perfluoro β-diketones (i .e., 1,1,1,2,2,6,6,6-octafluoro-3,5-hexadenione and 1,1,1,2,2,3,3,7,7,7-decafluoro-4,6-heptanedione. However, these are not separated in this distillation stage and are collected as one fraction composed of a mixture of two isomers. Each enolether is collected as one main fraction BPT 165-180 °C.

The yields and analytical data are reported in Table 1.

TABLE 1

Yields and Analytical Data for Silylenol Ethers

| Starting β-Diketone | Silylenol Ether Formed | Isolated Yield | Boiling Point | NMR |
|---|---|---|---|---|
| 1,1,1,5,5,5-hexafluoro-2,4-pentane dione | 4-(t-butyldimethylsiloxy)-1,1,1,5,5,5-hexafluoro-3-penten-2-one | 64% | 165-175°C | $^1$H $CDCl_3$ δ0.32(S,6H);δ0.99(S,9H);δ6.27(S,1H)<br>$^{13}$C $CDCl_3$ δ-4.34(S,2C);δ19.20(S,1C)δ25.51(S,3C);δ99.33(S,1C)δ116.0(Q,1C);δ120(Q,1C);δ156.1(Q,1C);δ178(Q,1C)<br>$^{19}$F $CDCl_3$ δ-76.5(S,3F);-70.2(S,3F) |
| 1,1,1,5,5,6,6,6-octafluoro-2,4-hexanedione | 4-(t-butyldimethylsiloxy)-1,1,1,5,5,6,6,6-octafluoro-3-hexen-2-one<br>and<br>2-(t-butyldimethylsiloxy)-1,1,1,5,5,6,6,6-octafluoro-2-hexen-4-one (ie. two isomers) | 62%, | 165-175°C | $^1$H $CD_2Cl_2$ δ0.35(S,9H);δ1.0(S,6H);δ6.4(S,1H)<br>(only major isomer shown) |
| 1,1,1,5,5,6,6,7,7,7-decafluoro-2,4-heptanedione | 4-(t-butyldimethylsiloxy)-1,1,1,5,5,6,6,7,7,7-decafluoro-3-hepten-2-one<br>and<br>2-(t-butyldimethylsiloxy)-1,1,1,5,5,6,6,7,7,7-2-hepten-4-one (i.e., two isomers) | 49%, | 165-180°C | $^1$H $CD_2Cl_2$ δ0.35(S,9H);δ1.0(S,6H);δ6.4(S,1H)<br>(only major isomer shown) |

Example 2

Preparation of ligands $(H_2)$DODECA-F[EDA]. $(H_2)$HEXADECA-F[EDA]. $(H_2)$EiCOSA-F[EDA]. $(H_2)$DODECA-F-[PDA]

The above ligands are prepared by reaction of a diamine and a silylenolether of a perfluorinated β-diketone.

Under nitrogen a dry 100ml reaction flask fitted with a mechanical stirrer, addition funnel and rubber septum is charged with silylenolether (0.125 moles) and cooled to -78 °C. The addition funnel charged with diamine (0.0625 moles) dissolved in an equal volume of THF is added over 5 minutes to the stirring enolether. The mixture is then allowed to warm to room temperature where it is stirred for one further hour. During either the addition of the diamine or the warm up period a thick white precipitate of ligand forms. This solid is filtered off and washed with methanol, boiled down in fresh methanol until white crystals appear, then left to stand and cool. Filtration yields colorless needles. Yields of specific compounds are listed in Table 2.

TABLE 2

Analytical Data for Ligands (H2)DODECA-F[EDA], (H2)HEXADECA-F[EDA], (H2)EiCOSA-F[EDA], (H2)DODECA-F[PDA]

| Starting Enolether | Diamine | Ligand | Yield | MPt | Elemental Analysis | NMR |
|---|---|---|---|---|---|---|
| 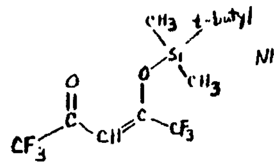 | $NH_2(CH_2)_2NH_2$ | (H2)DODECA-F[EDA] | 62% | 107°C | %calc for C12H8F12N2O2: C 32.74; H 1.83; F 51.79; N 6.36; O 7.27<br>Found: C 32.79; H 1.92; F 51.72; N 6.36 | $^1$H CDCl3 δ3.77(m,4H);δ5.87(S,2H); δ10.55(bs,2H)<br>$^{19}$F CDCl3 δ-66.2(S);δ-77.0(S)<br>$^{13}$C CDCl3 δ46.28;δ6.76;δ117.54; δ120.17;δ154.56;δ179.78 |
| 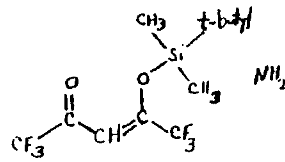 | $NH_2(CH_2)_3NH_2$ | (H2)DODECA-F[PDA] | 53% | 70-75°C | %calc for C13H10N2O2F12: C 34.38; H 2.22; N 6.17; O 7.05; F 50.19<br>Found: C 34.07; H 2.11; N 6.53; O ; F 45.6 | $^1$H CDCl3 δ2.07(p,2H);δ3.6(Q,4H); δ5.87(S,2H);δ10.5(bs,2H)<br>$^{19}$F CDCl3 δ-77.8(S);δ-67.4(S)<br>$^{13}$C CDCl3 δ-30.75(S);δ36.89(S) δ86.56(S);δ116.15(Q);δ119.0(Q); δ154.04(Q); δ180.07(Q) |
| 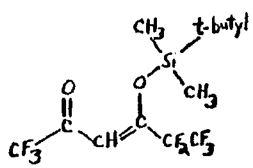 | $NH_2(CH_2)_2NH_2$ | (H2)HEXADECA-F[EDA] | 42% | 77-79°C | %calc for C14H8N2O2F16: C 31.13; H 1.49; N 5.19; O 5.92; F 56.27<br>Found: C 30.80; H 1.45; N 6.11; O ; F 54.3 | $^1$H CDCl3 δ3.75(t,2H);δ6.0(S,2H); δ10.6(bs,2H)<br>$^{19}$F CDCl3 δ-123.97(S);δ-82.81(S); δ-66.89(S)<br>$^{13}$C C6D6 δ44.18(S);δ88.30(S); δ108(tQ);δ118.88(Qt);δ119.13(Q); δ153.21(Q);δ182.26(t) |
| 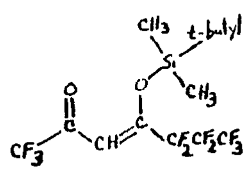 | $NH_2(CH_2)_2NH_2$ | (H2)EiCOSA-F[EDA] | 33% | 70-74°C | %calc for C16H8N2O2F20: C 30.02; H 1.25; N 4.38; O 5.00; F 59.35<br>Found: C 29.63; H 1.16; N 5.00; O ; F 53.3 | $^1$H CD3COCD3 δ4.1(S,4H);δ5.9(S,2H); δ10.7(bs,2H)<br>$^{19}$F CD3COCD3 δ-65.39(S); δ-80.15(t);δ-120.36(d); δ-126.31(S)<br>$^{13}$C CD3COCD3 δ46.36(S);δ88.08(S); δ109.97(tm);δ109.97(tt);δ112.94 (Qt);δ120.22(Q);δ154.49(Q) |

## Example 3

### Preparation of ligand $(H_2)$DODECA-F[POA]

Under a cover of nitrogen, a 100 ml reaction flask fitted with a reflux condenser, addition funnel and magnetic stir bar is charged with the t-butyl dimethylsilylenolether of hexafluoro-2,4-pentadione (9.66 g, 3 x $10^{-2}$ moles). The addition funnel is charged with 1,3-diamino-2-propanol (POA) (1.35g, 1.5 X $10^{-2}$ moles) in 5ml THF and this solution is then added over 5 mins, with stirring, to the enolether after which the reaction mixture is refluxed 15 hrs.

Upon cooling and standing overnight a buff colored solid forms which is filtered off and recrystallized from hexane/$CH_2Cl_2$ 50/50 to give colorless blocks. The yield was 6.5%. Analytical data is reported in Table 3 below.

**TABLE 3**

**Analytical Data for (H2)DODECA-F[POA]**

| Starting Enolether | Alkanolamine | Ligand Formed | NMR |
|---|---|---|---|
| 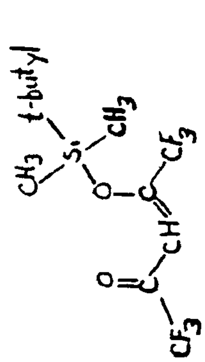 | 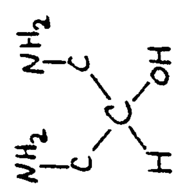 | (H2)DODECA-F[POA] | 'H CDCl3 δ2.3(bs,1H);δ3.6(m,4H); δ4.15(m,1H);δ5.9(S,2H); δ10.7(bs,2H) $^{19}$F CDCl3 δ-77.8(S);δ-67.4(S) $^{13}$C CDCl3 δ47.97(S);δ68.04(S); δ100.12(S);δ116.5(Q);δ118.93(Q); δ154.04(Q);δ180.0(Q) |

Example 4

Preparation of metal complexes of Perfluoro bis-(β-ketoimine) ligands

Under nitrogen, potassium methoxide (1.75 g, 0.025 moles) is dissolved in 150 ml dry methanol and 0.0125 mole of a bis-(β-ketoimine) ligand is added. The mixture is then stirred for 15 minutes to give a clear

bright yellow solution. Solid metal dibromide (0.0125 moles) is then added and the mixture stirred an additional 1 hour. The mixture is then filtered, the methanol evaporated off from the filtrate and the resultant solid redissolved in toluene (100 ml). This solution is filtered to remove residual potassium bromide and the filtrate evaporated to a solid that is then sublimed under a dynamic vacuum to yield the product complex. The analytical data is reported in Table 4 below:

TABLE 4

Analytical Data for perfluoro β-iminoketone and Bis-(perfluoro β-iminoketone) Metal Complexes

| Ligand | Metal Bromide | Complex | Yield | MPt | Mass Spectra | Elemental Analysis |
|---|---|---|---|---|---|---|
| (H2)DODECA-F[EDA] | CoBr2 | $Co^{+2}$DODECA-F[EDA] | 74.2% | 111°C | Cal 496.9570<br>Found 496.9533 | Calculated for: $C_{12}H_6N_2O_2F_{12}Co$<br>% C 28.99; H 1.22; N 5.63; O 6.44; F 45.86; Co 11.85<br>Found C 29.26; H 1.32; N 4.15; O ; F 41.40; Co 10.60 |
| (H2)DODECA-F[EDA] | CuBr2 | $Cu^{+2}$DODECA-F[EDA] | 92.3% | 132-135°C | Cal 500.9534<br>Found 500.9512 | Calculated for: $C_{12}H_6N_2O_2F_{12}Cu$<br>% C 28.73; H 1.21; N 5.58; O 6.38; F 45.44; Cu 12.67<br>Found C 28.85; H 1.20; N 4.03; O ; F 40.10; Cu 12.30 |
| (H2)HEXADECA-F[EDA] | CoBr2 | $Co^{+2}$HEXADECA-F[EDA] | 67% | 75-80°C | Cal 596.9506<br>Found 596.9511 | Calculated for: $C_{14}H_6N_2O_2F_{16}Co$<br>% C 28.16; H 1.01; N 4.69; O 5.36; F 50.91; Co 9.87<br>Found C 27.52; H 1.07; N 3.80; O ; F 46.20; Co 9.53 |
| (H2)HEXADECA-F[EDA] | CuBr2 | $Cu^{+2}$HEXADECA-F[EDA] | 87.6% | 112-120°C | Cal 600.9470<br>Found 600.9467 | Calculated for: $C_{14}H_6N_2O_2F_{16}Cu$<br>% C 27.94; H 1.01; N 4.65; O 5.32; F 50.52; Cu 10.56<br>Found C 28.25; H 0.88; N 3.98; O ; F 48.20; Cu 10.20 |
| (H2)EiCOSA-F[EDA] | CoBr2 | $Co^{+2}$EiCOSA-F[EDA] | 91.6% | 82-84°C | Cal 696.9442<br>Found 696.9450 | Calculated for: $C_{16}H_6N_2O_2F_{20}Co$<br>% C 27.57; H 0.87; N 4.02; O 4.59; F 54.50; Co 8.45<br>Found C 27.49; H 0.76; N 3.82; O ; F 49.50; Co 8.09 |
| (H2)EiCOSA-F[EDA] | CuBr2 | $Cu^{+2}$EiCOSA-F[EDA] | 90.1% | 99-102°C | Cal 700.9406<br>Found 700.9437 | Calculated for: $C_{16}H_6N_2O_2F_{20}Cu$<br>% C 27.39; H 0.86; N 3.99; O 4.56; F 54.15; Cu 9.06<br>Found C 27.62; H 0.72; N 3.67; O ; F 55.40; Cu 8.70 |

Example 5

Relative volatility measurement of perfluoro β-ketoiminato complexes

A standard weight loss experiment for each metal complex was conducted by heating a sample of approximately 50 mg at 10° C/min under a 100 cc/min flow of nitrogen using a DuPont Model No. 951. Thermal Gravimetric Analyzer in conjunction with a model No. 9900 Controller. Table 5 below indicates that all of the metal complexes tested in this way are clearly volatile, leaving as little as 0.641% residue at the end of an evaporation cycle. The data reported indicates that the evaporation of these complexes is a smooth process, i.e. a gradual and even transition from the solid to the gas phase.

TABLE 5

Volatility of Perfluoro bis-(β-ketoiminato) Copper Complexes

| Complex | Temperature T°C at which complex is completely vaporized | Residual weight left at T°C |
|---|---|---|
| $Cu^{+2}$DODECA-F[EDA] | 245 | 1.07% |
| $Cu^{+2}$HEXADECA-F[EDA] | 245 | 1.94% |
| $Cu^{+2}$EICOSA-F[EDA] | 245 | 0.641% |

Example 6

A run was carried out in an attempt to reproduce the work of Richardson and Sievers for the synthesis of the ligand

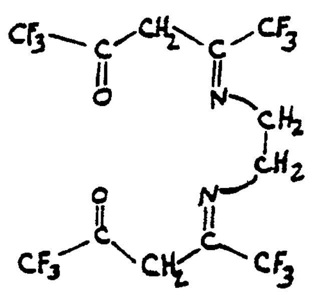

as described in J.Inorg. Nucl. Chem. 1970, vol. 32, p. 1895 to 1906.

4.16g (0.02 moles) of 1,1,1,5,5,5-hexafluoro-2,4-pentanedione were dissolved in 10 ml of benzene and to this solution 0.60g (0.01 mole) of ethylenediamine dissolved in 10 ml benzene were slowly added, with stirring, over a period of five minutes. A cream colored precipitate slowly formed and heat was evolved. The mixture was then boiled and filtered hot. The resulting white precipitate was then washed with hot benzene, refiltered and left to air dry. Yield = 4.4g white solid. To check if any ligand may have formed at this point, the white solid was analyzed by Gas Chromatography (using a Hewlett Packard 5880A gas chromatograph). In this determination an acetone solution of the white salt was shown to contain none of the desired ligand (by comparison to results obtained for the analysis of pure authentic ligand; i.e., $H_2$-DODECA-F[EDA] as obtained via the silylenolether route as described in this patent disclosure). The white salt was then heated under vacuum in a sublimator to 90° C whereupon the apparatus was sealed shut and the sublimation process allowed to occur. The temperature of the cooling water was set at 16° C. After two hours, a fine powdery film had formed upon the cold finger of the sublimator. This did not bear a strong resemblance to the "large shiny white feathery needles" described by Richardson and Sievers. Gas chromatographic analysis of this solid film showed that it contained no $H_2$-DODECA-F[EDA] ligand. The cold finger of the sublimator was then cleaned, the apparatus reassembled and the sublimation process reinitiated under the

same conditions as before allowing two hours of sublimation time. Again, a white film appeared on the cold finger. Gas chromatographic analysis of this solid indicated a total absence of $H_2$-DODECA-F[EDA] ligand.

Having thus described the present invention, what is now deemed appropriate for Letters Patent in set out in the following appended claims.

## Claims

1. A thermally volatilizable, β-ketoiminato metal complex having the structural formula:

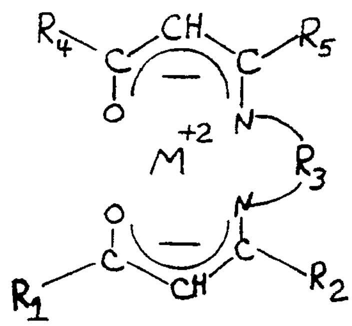

wherein $R_1$, $R_2$, $R_4$ and $R_5$ are independently linear or branched, perfluorinated, $C_1$-$C_8$ alkyl groups, $R_3$ is an unfluorinated, partially fluorinated or fully fluorinated phenylene or $C_1$-$C_8$ alkylene or hydroxyalkylene group and $M^{+2}$ is a divalent metal ion.

2. A metal complex in accordance with Claim 1 wherein $R_1$, $R_2$, $R_4$ and $R_5$ are $CF_3$.

3. A metal complex in accordance with Claim 1 wherein $R_1$ and $R_2$ are independently perfluorinated methyl, ethyl or propyl groups.

4. A metal complex in accordance with Claim 3 wherein $R_3$ is $CH_2CH_2$.

5. A metal complex in accordance with Claim 1 wherein $M^{+2}$ is $Cu^{+2}$.

6. A metal complex in accordance with Claim 1 wherein $M^{+2}$ is $Co^{+2}$.

European Patent Office

# EUROPEAN SEARCH REPORT

Application Number

EP 91103405.6

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| P,D,<br>X | EP - A2 - 0 373 513<br>(AIR PRODUCTS AND CHEMICALS)<br>* Claims 1-6,10-17 *<br>-- | 1-6 | C 07 C 251/04//<br>//C 23 C 16/18 |
| P,X | EP - A1 - 0 369 297<br>(AIR PRODUCTS AND CHEMICALS)<br>* Claims 1,4,5,10,13,14,16,<br>17 *<br>-- | 1-3,5,<br>6 | |
| X | US - A - 3 594 216<br>(ROBERT G. CHARLES et al.)<br>* Claims 1-3 *<br>-- | 1-6 | |
| A | US - A - 3 903 118<br>(PRITAM SINGH DHALIWAL)<br>* Claim 1 *<br>-- | 1 | |
| A | US - A - 3 388 141<br>(MORRIS B. BERENBAUM)<br>* Claims 1,4 *<br>---- | 1 | |

TECHNICAL FIELDS SEARCHED (Int. Cl.5)

C 07 C 251/00
C 07 C 225/00
C 07 F
C 23 C

The present search report has been drawn up for all claims

| Place of search | Date of completion of the search | Examiner |
|---|---|---|
| VIENNA | 07-06-1991 | REIF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document